Europäisches Patentamt

(19) European Patent Office

Office européen des brevets

(11) Publication number: **0 236 088**
**A2**

(12) # EUROPEAN PATENT APPLICATION

(21) Application number: **87301746.1**

(22) Date of filing: **27.02.87**

(51) Int. Cl.⁴: **A 01 C 1/00**

(30) Priority: **01.03.86 JP 44773/86**

(43) Date of publication of application:
**09.09.87 Bulletin 87/37**

�">Designated Contracting States: **DE FR GB**

(71) Applicant: **SHIMAZAKI SEED CO., LTD.**
**74, Shinmachi**
**Hamamatsu-shi Shizuoka (JP)**

(72) Inventor: **Shimazaki, Fujio**
**740-1, Tennocho**
**Hamamatsu-shi Shizuoka (JP)**

(74) Representative: **Ellis, Edward Lovell et al**
**MEWBURN ELLIS & CO. 2/3 Cursitor Street**
**London EC4A 1BQ (GB)**

(54) A method of preparing magnetized seeds.

㉗ A method for improving plant yield, acceleration plant growth, and accelerating germination of seeds by exposing the seeds to a magnetic field along with a treatment using a ferrous ingredient.

EP 0 236 088 A2

## Description

<u>A METHOD OF PREPARING MAGNETIZED SEEDS</u>

This invention relates to a magnetization of vegetable seeds.

There has hitherto been widely utilized a magnetic field for improving a human physiology. The applicant has invented, on the other hand, a method of preparing magnetized seeds and filed the patent application therefor (Japanese Patent Application No. 59-21006). Subsequently, the applicant has further developed the method by use of an electrolytic ferrous ion for enhancing the magnetization thereby to increase a physiological activity of vegetables. The preparation of the electrolytic ferrous ion, however, has a problem in a view-point of equipment and cost. The applicant has therefore continued the study for searching an activating ingredient and has now found out that a solution of a ferrous salt has more or less the same effect as the electrolytic ferrous ion.

It is sought by magnetizing vegetable seeds to activate a composition of the seeds, reduce a growth period of the seeds and enhance a reproduction of the seeds.

The invention provides a method of preparing magnetized seeds, which comprises the steps of:
sterilizing the seeds for a predetermined period of time,
washing the sterilized seeds with water,
dipping the seeds thus treated into a solution of an activating ingredient for a predetermined period of time;
drying the seeds; and
magnetizing the seeds by means of a magnet or electromagnet under a magnetic flux of at least 1 gauss for a predetermined period of time.

In one embodiment of the invention, the dipping treatment may be carried out in a 50 to 100 fold solution of an electrolytic ferrous ion for more than 12 hours, and the magnetization may be carried out under a magnetic flux of at least 500 gauss for $10 \pm 5$ minutes. Further, in this embodiment, the solution of the electrolytic ferrous ion preferably contains as a chemical element a ferrous ion ($Fe^{++}$) of a cathodic liquid which has been prepared by electrolysis with a metal iron cathode, for example at 0.1A for 20 minutes in an electrolytic vessel containing 0.1% $H_2SO_4$ and separated by a glass wool membrane.

In another embodiment of the invention, the dipping treatment may be carried out in a solution of a ferrous salt before the magnetization under the magnetic flux of at least 1 gauss. Alternatively, the magnetization may be carried out under the magnetic flux of at least 1 gauss before the dipping treatment in the solution of the ferrous salt. In this embodiment, the ferrous salt may consist of ferrous sulfate or ammonium ferrous sulfate in the form of a 0.5 to 2% solution, into which the seeds are dipped for 10 to 15 hours, and the magnetization is preferably carried out for $10 \pm 5$ minutes. Further, in this embodiment, the water for dissolving the salt is desirably boiled previously for removing dissolved oxygen in order to prevent the ferrous salt from being converted by oxidation to a ferric salt.

In accordance with the invention, by the magnetization along with the treatment using the activating ingredient, such as the electrolytic ferrous ion or the ferrous salt, there have been observed several advantages including the activation of vegetable cells, the improved photo-synthesis under a less amount of light, the promotion of germination and growth, as well as the increased yield.

There has hitherto been known that the growth of vegetables, especially leaves and roots has a specific orientation relative to the terrestrial magnetism, and that the orientation of roots has an influence on sexual determination. Further, it has been known that the orientation of young roots toward the north pole may produce a larger number of female flowers than the orientation toward the south pole, while the orientation of the young roots toward the south may promote the germination and growth. Accordingly, the magnetization of seeds containing a certain ferrous ingredient may promote the growth of vegetables and increase the number of female flowers. Further, the activation of vegetable cells, the improved photo-synthesis in the twilight, the stabilization and dramatic increase of crop yield may be ensured.

The several embodiment for magnetizing the seeds has been described herein-above and will be defined in the subclaims 2 to 7.

For better understanding, the invention will be described with the following Examples.

[Example 1]

A 50 - 100 fold solution of an electrolytic ferrous ion was prepared by electrolysis using a metal iron as a cathode at a 0.1A for 20 minutes in an electrolytic vessel containing 0.1% $H_2SO_4$ and separated by a glass wool membrane to form a colorless solution containing as a chemical element a stable ferrous ion ($Fe^{++}$) in a solvent (water).

[Example 2]

Seeds of kidney beans were cultivated in a green-house for comparing the magnetized seed of the invention with non-treated normal seed.

The seed of the invention was immersed in a 100 fold solution of the ferrous ion overnight (12 hours) before working.

Condition : Temperature in House 20°C
Earth Temp. in House 20°C

0 236 088

Variety 'KUROKINTOKI'
The seed was sown on October 10.

| MAGNETIZED SEED | | | |
|---|---|---|---|
| Germination No. of Days | Days till Harvest | Flowering Figures | Harvested Legume Figures |
| 3 days | 34 days | 63/per stock | 22/per stock |

| NORMAL SEED | | | |
|---|---|---|---|
| Germination No. of Days | Days till Harvest | Flowering Figures | Harvested Legume Figures |
| 5 days | 51 days | 41/per stock | 10/per stock |

[Example 3]
    Green soybean seeds were magnetized as above, and were cultivated in a greenhouse with non-treated control seeds.
    The seed was immersed in a 50 fold solution of the ferrous ion overnight (12 hours) before working.
    Condition : Temperature in House 25°C
Earth Temp. in House 25°C
Variety 'KOFURISODE'
    The seed was sown on October 10.

3

| MAGNETIZED SEED | | | |
|---|---|---|---|
| Germination No. of Days | Days till Harvest | Flowering Figures | Harvested Legume Figures |
| 8 days | 65 days | 95/per stock | 37/per stock |

| NORMAL SEED | | | |
|---|---|---|---|
| Germination No. of Days | Days till Harvest | Flowering Figures | Harvested Legume Figures |
| 11 days | 83 days | 75/per stock | 22/per stock |

As can be seen from Examples 2 and 3, the magnetized seed germinated more quickly than the unmagnetized seed, was ready for harvest more quickly, and provided more flowering figures and harvested legume figures.

[Example 4]

Preparation of a ferrous salt solution

Into a sealed vessel equipped with an upper inlet for a $N_2$ gas and a discharge tube was introduced 1000 ml of water, which has previously been boiled for removing dissolved oxygen, and then 20g of ferrous sulfate ($FeSO_4.7H_2O$) was added and dissolved uner a $N_2$ blanket. Then, the desired seed was placed therein.

[Example 5]

Seeds of kidney beans were cultivated in a green-house for comparing the magnetized seed of the invention with non-treated normal seed.

The seed of the invention was immersed in a solution of ferrous sulfate overnight (12 hours) before working.

Condition : Temperature in House 20°C

Earth Temp. in House 20°C

Variety 'KUROKINTOKI"

The seed was sown on October 10.

| MAGNETIZED SEED | | | |
|---|---|---|---|
| Germination No. of Days | Days till Harvest | Flowering Figures | Harvested Legume Figures |
| 3 days | 35 days | 64/per stock | 25/per stock |

| NORMAL SEED | | | |
|---|---|---|---|
| Germination No. of Days | Days till Harvest | Flowering Figures | Harvested Legume Figures |
| 5 days | 50 days | 40/per stock | 9/per stock |

[Example 6]

Green soybean seeds were magnetized as above, and were cultivated in a greenhouse with non-treated control seeds.

The seed of the invention was immersed in a solution of ferrous sulfate overnight (12 hours) before working.

Condition : Temperature in House 25°C

Earth Temp. in House 25°C

Variety 'KOFURISODE"

The seed was sown on October 10.

| MAGNETIZED SEED | | | |
|---|---|---|---|
| Germination No. of Days | Days till Harvest | Flowering Figures | Harvested Legume Figures |
| 8 days | 64 days | 94/per stock | 35/per stock |

| NORMAL SEED | | | |
|---|---|---|---|
| Germination No. of Days | Days till Harvest | Flowering Figures | Harvested Legume Figures |
| 11 days | 85 days | 75/per stock | 21/per stock |

As can be seen from Example 5 and 6, the magnetized seed germinated more quickly than the unmagnetized seed, was ready for harvest more quickly, and provided more flowering figures and harvested legume figures.

It can be seen from the above examples that the magnetized seeds exhibited accelerated growth and reproductivity, resulting in increased yields. It is postulated that the magnetized seeds provided superior plants because the iron, an important mineral constituent of the seed, was activated to an extent to accelerate growth.

Among the advantages of using magnetized seed are improved plant freshness and longer lasting crops after harvest.

From the mid-point of growth of plants from magnetized seeds, the arbor vivacity flourishes. Under low temperature conditions, even with abundant rain and excessively wet soil, the pistillate flower and pollen volume of the staminate flower are in abundance, with no seed shedding after pollination. Compared with untreated seeds, the growth is noticeably accelerated.

**Claims**

1. A method of preparing magnetized seeds, which comprises the steps of:
sterilizing the seeds for a predetermined period of time,
washing the sterilized seeds with water,
dipping the seeds thus treated into a solution of an activating ingredient for a predetermined period of time,
drying the seeds; and
magnetizing the seeds by means of a magnet or electromagnet under a magnetic flux of at least 1 gauss for a predetermined period of time.

2. A method according to claim 1, wherein the dipping treatment is carried out in a 50 to 100 fold solution of an electrolytic ferrous ion for at least 12 hours, and the magnetization is carried out under a magnetic flux of at least 500 gauss for $10 \pm 5$ minutes.

3. A method according to claim 2, wherein the solution of the electrolytic ferrous ion contains as a chemical element a ferrous ion of a cathodic liquid which has been prepared by electrolysis with a metal iron cathode.

4. A method according to claim 1, wherein the dipping treatment is carried out in a solution of a ferrous salt before the magnetization under the magnetic flux of at least 1 gauss.

5. A method according to claim 1, wherein the magnetization is carried out under the magnetic flux of at least 1 gauss before the dipping treatment in a solution of a ferrous salt.

6. A method according to claim 4 or 5, wherein the ferrous salt consists of ferrous sulfate or ammonium ferrous sulfate in the form of a 0.5 to 2% solution, into which the seeds are dipped for 10 to 15 hours.

7. A method according to claim 4 or 5, wherein the magnetization is carried out for $10 \pm 5$ minutes.

8. A method according to claim 3 wherein the electrolysis with the metal iron cathode is carried out at about 0.1A for about 20 minutes in about 0.1% $H_2SO_4$.